# EUROPEAN PATENT APPLICATION

(11) **EP 4 787 344 A1**
(43) Date of publication of application: **05.08.2026**
(21) Application number: 24872969.1
(22) Date of filing: 26.09.2024
(51) Int. Cl.: G07C 9/00, G07C 9/29, G07C 9/27, G06N 20/00, G16H 10/60

(54) **DEVICE FOR CONTROLLING OPENING AND CLOSING OF ENTRANCE DOOR BY USING ARTIFICIAL INTELLIGENCE AND METHOD FOR OPENING AND CLOSING ENTRANCE DOOR**

(30) Priority: 26.09.2023 KR 20230129384; 29.12.2023 KR 20230196436
(71) Applicant: Won International Inc., Dong-gu, Gwangju 61438 (KR)
(72) Inventor: BYEON, Cheol Won, 61011 Gwangju (KR)
(74) Representative: Jung, Minkyu
(86) International application number: PCT/KR2024/014600
(87) International publication number: WO 2025/071261

(57) **Abstract**

The present invention relates to a method for controlling opening and closing of a door, which may comprise: receiving at least one of image information generated by a photographing device and user authentication information around the door; determining a user approaching the door based on at least one of the image information and the user authentication information; transmitting a door lock unlocking signal to a door lock device in response to determining that the user is an authenticated user; and transmitting a door opening signal to a door opening/closing device as the door lock device releases the lock of the door in response to the door lock unlocking signal.

## Description

### BACKGROUND

### Field

The present invention relates to an apparatus for controlling opening and closing of a door using artificial intelligence and a method for opening and closing a door, and more particularly, to an apparatus, method, and system for detecting a person approaching an automatic door based on artificial intelligence, identifying the person, determining whether the identified person is an authenticated person, releasing a lock of a door lock, and opening the automatic door.

### Related Art

Most commercially available electronic door locks and door opening/closing systems are incompatible with each other, causing inconvenience in use.

For example, conventional door locks used in private residences are specialized for locking and unlocking a locking mechanism and do not have a function of opening and closing the door itself. In addition, devices referred to as automatic door systems are also incompatible with commercially available digital door locks, and in most cases, separate locking devices are used in addition to the simple function of opening and closing the door.

This requires a procedure of releasing the locking mechanism of the electronic door lock when opening or closing the door after the release, which is cumbersome.

In such a situation, when a general person is unable to use their hands freely-for example, because they are carrying something or using a stroller-entering and exiting through a door is not only inconvenient, but is also extremely difficult for persons with disabilities or users of mobility aids.

Therefore, there is a need for a system capable of analyzing signals from existing electronic door locks and automatically opening and closing a door by recognizing a user.

### SUMMARY

The present invention is directed to solving the problems of the prior art described above. An object of the present invention is to detect a person entering or exiting using artificial intelligence while being linked with a door lock and a door, determine whether the person is an authenticated user, release the door lock, and automatically open and close the door.

A method for controlling opening and closing of a door according to the present invention for achieving the above object may include: receiving at least one of image information generated by a photographing device and user authentication information around the door; determining a user approaching the door based on at least one of the image information and the user authentication information; transmitting a door lock unlocking signal to a door lock device in response to determining that the user is an authenticated user; and transmitting a door opening signal to a door opening/closing device as the door lock device releases the lock of the door in response to the door lock unlocking signal.

Here, the step of determining a user approaching the door may include detecting, based on an artificial intelligence model, a user approaching the door from the image information, and determining whether the detected user is an authenticated user.

In addition, the step of determining a user approaching the door may include detecting, based on an artificial intelligence model, a user approaching the door from the image information, and determining a disability type associated with the detected user based on the image information.

Further, the door opening/closing device may be controlled to open and close the door differently based on the disability type associated with the user.

Furthermore, the disability type may include at least one disability type, and the speed at which the door opening/closing device opens and closes the door may be controlled differently based on the user corresponding to the at least one disability type.

In addition, the at least one disability type may include a first type through a third type, wherein the first type controls the door opening/closing device to open and close the door at a first speed, the second type controls the door opening/closing device to open the door more slowly than the first speed and to close the door more slowly than the first speed, and the third type controls the door opening/closing device to open the door faster than the first speed and to close the door faster than the first speed.

Also, the at least one disability type may include a first type through a third type, wherein the first type controls the door opening/closing device to open the door to a first angle, the second type controls the door opening/closing device to open the door to a second angle greater than the first angle, and the third type controls the door opening/closing device to open the door to a third angle greater than the second angle.

In addition, the disability type may include at least one of a visually impaired person, a person with a disability riding a mobility aid, and a person with a disability using crutches.

Furthermore, the state associated with the user may include a state in which both hands of the user are occupied by a specific object, and in response to determining from the image information that the user is in a state in which it is impossible to use their hands, the lock of the door lock device may be released and the door opening/closing device may be controlled to open the door.

In addition, the user authentication information may include at least one of biometric information associated with the user registered in a user terminal, an authentication key, device authentication information, and an RFID signal.

Furthermore, the step of transmitting the door opening signal to the door opening/closing device as the door lock device releases the lock of the door may further include transmitting the door opening signal to the door opening/closing device after receiving a signal from the door lock device indicating that the unlocking of the door lock is complete.

In addition, the method may further include transmitting a door closing signal for closing the door to the door opening/closing device in response to detecting that the user has entered or exited through the door after the door is opened.

Also, the method may further include transmitting a door locking signal for switching the door to a locked state to the door lock device in response to detecting that the door is closed.

Meanwhile, an apparatus for controlling opening and closing of a door according to the present invention for achieving the above object may include: an information transceiver configured to receive at least one of image information generated by a photographing device around the door and user authentication information; a user authentication unit configured to determine a user approaching the door based on at least one of the image information and the user authentication information; and a controller configured to transmit a door lock unlocking signal to a door lock device in response to determining that the user is an authenticated user, and to transmit a door opening signal to a door opening/closing device as the door lock device releases the lock of the door in response to the door lock unlocking signal.

Meanwhile, a system for controlling opening and closing of a door according to the present invention for achieving the above object may include: a photographing device located around the door and configured to photograph a user approaching the door; a door opening/closing device connected to the door and configured to open and close the door; a door lock device installed on one side of the door and configured to lock or unlock the door; and a control device configured to receive image information generated by the photographing device, determine a user approaching the door based on at least one of the image information and the user authentication information, transmit a door lock unlocking signal to the door lock device in response to determining that the user is an authenticated user, and transmit a door opening signal to the door opening/closing device as the door lock device releases the lock of the door in response to the door lock unlocking signal.

The device for controlling opening and closing of a door according to the present invention has the following effects.

First, a user can easily enter and exit through a door. The apparatus for controlling opening and closing of a door according to the present invention authenticates a user using artificial intelligence, and automatically releases the door lock and opens the door when the authenticated user approaches the door. Accordingly, even a physically impaired user can easily enter and exit through the door.

Second, security can be enhanced. The apparatus for controlling opening and closing of a door according to the present invention releases the door lock and opens the door when an authenticated user approaches, and does not release the door lock when an unauthenticated user approaches. Accordingly, since the door lock is released only when an authenticated user enters or exits, persons with malicious intent can be prevented from entering, thereby enhancing security.

### BRIEF DESCRIPTION OF THE DRAWINGS

FIG. 1 is a block diagram of a system for controlling opening and closing of a door according to an embodiment of the present invention.
FIG. 2 is a block diagram of an apparatus for controlling opening and closing of a door according to an embodiment of the present invention.
FIG. 3 is a flowchart of a method for controlling opening and closing of a door according to an embodiment of the present invention.
FIG. 4 is a diagram exemplarily illustrating detection of a user approaching a door in the apparatus for controlling opening and closing of a door according to an embodiment of the present invention.
FIG. 5 is a diagram exemplarily illustrating detecting a user and determining whether the user is an authenticated user in the apparatus for controlling opening and closing of a door according to an embodiment of the present invention.
FIG. 6 is a diagram exemplarily illustrating determining an authenticated user, releasing the door lock, and opening the door in the apparatus for controlling opening and closing of a door according to an embodiment of the present invention.
FIG. 7 is a diagram exemplarily illustrating detecting a person with a disability and controlling the door to open in the apparatus for controlling opening and closing of a door according to an embodiment of the present invention.

### [DESCRIPTION OF REFERENCE NUMERALS]

100: door opening/closing device
200: photographing device
300: door lock device
400: control device
410: information transceiver
420: user authentication unit
430: controller

### DESCRIPTION OF EXEMPLARY EMBODIMENTS

Specific structural or functional descriptions of embodiments according to the concept of the present invention disclosed in the present specification are merely exemplary for the purpose of describing the embodiments according to the concept of the present invention, and the embodiments according to the concept of the present invention may be implemented in various forms and are not limited to the embodiments described in the present specification.

Since the embodiments according to the concept of the present invention can be subjected to various changes and can take various forms, the embodiments are illustrated in the drawings and described in detail in the present specification.

However, this is not intended to limit the embodiments according to the concept of the present invention to specific disclosed forms, and includes all changes, equivalents, or substitutes included in the spirit and technical scope of the present invention.

When an element is referred to as being "connected" or "coupled" to another element, it should be understood that the element may be directly connected or coupled to the other element, but another element may also exist in between. On the other hand, when an element is referred to as being "directly connected" or "directly coupled" to another element, it should be understood that no other element exists in between.

The terminology used herein is used only for the purpose of describing particular embodiments and is not intended to limit the present invention.

Singular expressions include plural expressions unless the context clearly dictates otherwise. In the present specification, terms such as "comprise" or "have" are intended to designate that the described features, numbers, steps, operations, elements, components, or combinations thereof exist, and should not be understood as precluding the possibility of the presence or addition of one or more other features, numbers, steps, operations, elements, components, or combinations thereof.

Unless otherwise defined, all terms used herein, including technical or scientific terms, have the same meaning as commonly understood by one of ordinary skill in the art to which the present invention belongs.

Terms defined in commonly used dictionaries should be interpreted as having a meaning consistent with the meaning in the context of the relevant technology, and unless clearly defined in the present specification, should not be interpreted in an ideal or overly formal sense.

Hereinafter, specific technical configurations of the present invention will be described with reference to the accompanying drawings.

### <System for Controlling Opening and Closing of a Door>

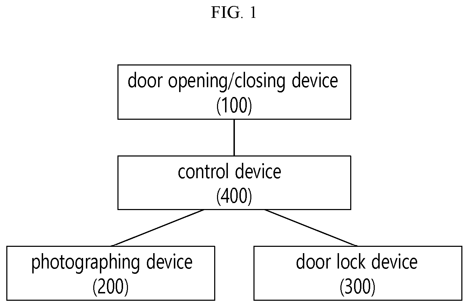

FIG. 1 is a block diagram of a system for controlling opening and closing of a door according to an embodiment of the present invention.

As shown in FIG. 1, the system for controlling opening and closing of a door may include a door opening/closing device 100, a photographing device 200, a door lock (Doorlock) device 300, and a control device 400.

The door opening/closing device 100 is installed on a door and is a component that opens and closes the door installed at an entrance of a building so that a user can enter and exit the interior and exterior of the building through the door. The door opening/closing device 100 can open and close the door so that an authenticated user can enter and exit the interior and exterior of the building.

The door opening/closing device 100 is installed on the interior, exterior, hinge, door frame, or wall of the door, and can calculate the material, weight, and wind pressure of the door and open and close the door upon receiving a door opening signal or a door closing signal.

In addition, a motor is provided on one side of the door so that the door can be opened and closed in a sliding manner or a hinged manner by the power provided by the motor. Accordingly, the door opening/closing device 100 can control the motor to open and close the door in a sliding manner or a hinged manner.

Here, after the door is opened, the door opening/closing device 100 can detect whether an authenticated user has entered or exited through the door based on an entry sensor (not shown) installed on one side of the door, and can transmit an entry detection signal to the control device 400. In addition, the sensor can detect when the door is closed and transmit a door closing signal to the control device 400.

The photographing device 200 is a component that photographs a user approaching the door to generate image information and transmits the generated image information to the control device 400. At least one photographing device 200 may be installed at the door itself or at the upper left or upper right of the surroundings of the door.

The door lock device 300 is a component that locks or unlocks the door. When it is determined that a user approaching the door is an authenticated user and a door lock unlocking signal related to the door lock is received, the door lock device 300 can release the lock of the door lock with respect to the door opening/closing device 100, and when the door lock unlocking signal related to the door lock is not received, the door lock device 300 may not release the lock of the door lock with respect to the door opening/closing device 100.

The control device 400 is connected to the door opening/closing device 100, the photographing device 200, and the door lock device 300, and is a component that determines a user approaching the door, releases the lock of the door lock with respect to the door accordingly, and controls the door to open. The control device 400 may be implemented integrally with the door lock device 300, may be implemented as being installed adjacent to the door lock device 300 on any one part of the door, or may be implemented as being connected to the door opening/closing device 100 and the door lock device 300 via a network at a location separate from the door.

In addition, the control device 400 can receive image information including a user approaching the door from the photographing device 200, and determine whether the user in the received image information is an authenticated user.

Furthermore, if the user included in the image information is an authenticated user, the control device 400 can transmit a door lock unlocking signal related to the door lock to the door lock device 300 to control the lock related to the door lock that maintains the door in a locked state to be released.

Moreover, the control device 400 can receive a signal from the door lock device 300 indicating that the unlocking of the door lock is complete, and then transmit a door opening signal to the door opening/closing device 100 to control the door to open.

In addition, if it is determined that the authenticated user has entered or exited through the door, the control device 400 can transmit a door closing signal to the door opening/closing device 100 to control the door to close.

Also, if it is determined that the door is closed, the control device 400 can transmit a door locking signal to the door lock device 300 to control the door lock to lock the door again.

The control device 400 can register and store user information about authenticated users. Such user information may be registered including the user's body, face, presence or absence of disability, and characteristics.

### <Configuration of the Control Device>

Hereinafter, the control device 400 that controls the opening and closing of the door and controls the locking and unlocking of the door lock will be described with reference to the drawings.

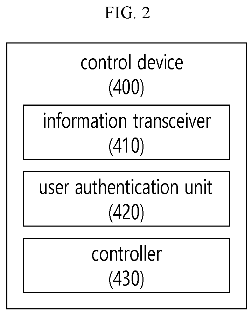

FIG. 2 is a block diagram of an apparatus for controlling opening and closing of a door according to an embodiment of the present invention.

As shown in FIG. 2, the apparatus for controlling opening and closing of a door according to an embodiment of the present invention may include an information transceiver 410, a user authentication unit 420, and a controller 430.

The information transceiver 410 is a component that receives information from external components of the control device 400. The information transceiver 410 can transmit and receive information to and from the door opening/closing device 100, the photographing device 200, the door lock device 300.

For example, the information transceiver 410 can receive image information obtained by photographing the front of the door from the photographing device 200.

In addition, the information transceiver 410 can transmit a door lock unlocking signal related to the door lock to the door lock device 300.

Also, the information transceiver 410 can receive a signal from the door lock device 300 indicating that the unlocking of the door lock is complete, and can transmit a door opening signal to the door opening/closing device 100.

Furthermore, the information transceiver 410 can receive an entry detection signal detected by an entry sensor installed on one side of the door indicating whether an authenticated user has entered or exited through the door.

Also, the information transceiver 410 can receive a door closing signal detected by the sensor when the door is closed.

The user authentication unit 420 is a component that identifies a user included in image information and determines whether the user is an authenticated user. The user authentication unit 420 can be implemented as artificial intelligence to determine whether a user included in image information is an authenticated user. The user authentication unit 420 can identify and determine a user from image information based on accumulated data about the user.

The controller 430 is a component that controls the door to open depending on whether a user approaching the door is an authenticated user. If the user authentication unit 420 determines that the user approaching the door is an authenticated user, the controller 430 can transmit a door lock unlocking signal related to the door lock to the door lock device 300 to control the door lock to be released.

In addition, when the controller 430 receives a signal from the door lock device 300 indicating that the unlocking of the door lock is complete, the controller 430 can transmit a door opening signal to the door opening/closing device 100 to control the door to open.

Also, when the entry of the authenticated user through the door is confirmed via the door opening/closing device 100, the controller 430 can transmit a door closing signal to the door opening/closing device 100 to control the door to close.

Also, when the controller 430 receives a signal indicating that the closing of the door is complete, the controller 430 can transmit a door locking signal to the door lock device 300 to control the door lock to lock the door again.

### <Method for Controlling Opening and Closing of a Door>

Hereinafter, a method for controlling opening and closing of a door will be described with reference to the drawings.

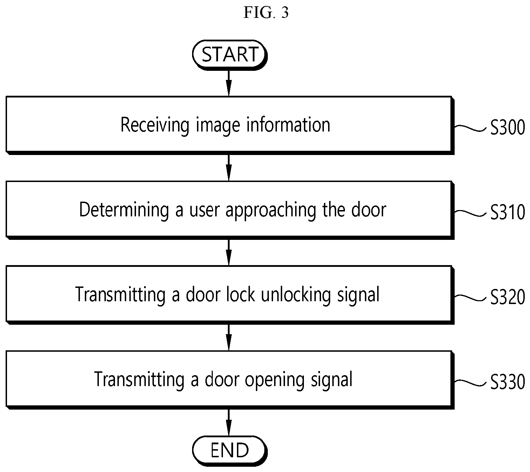

FIG. 3 is a flowchart of a method for controlling opening and closing of a door according to an embodiment of the present invention.

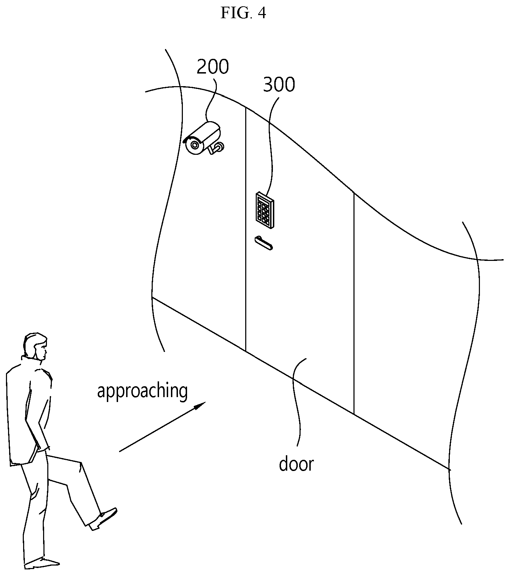

As shown in FIG. 3, the method for controlling opening and closing of a door according to an embodiment of the present invention may first receive image information generated by the photographing device 200 around the door. <S300> This will be described with reference to FIG. 4.

FIG. 4 is a diagram exemplarily illustrating detection of a person approaching a door in the apparatus for controlling opening and closing of a door according to an embodiment of the present invention.

As shown in FIG. 4, a user or person inside or outside a specific building may approach the door to enter the interior of the building. Conversely, a user or person may approach the door to exit to the exterior of the building.

Here, a user may be a person who has accessed the control device 400 through a terminal and is approaching the door, and a person may refer to anyone approaching the door, including a user, regardless of whether they have accessed the control device 400. In the present invention, a user may be interpreted to have the same meaning as a person, as simply a person passing through the door.

At this time, the photographing device 200 installed around the door can photograph a user approaching the door to generate image information, and transmit the generated image information to the control device 400.

For example, the photographing device 200 may be installed near the upper portion of the outer surface of the door facing toward the outside of the building to photograph a user approaching from the front of the door, and transmit the photographed image information to the control device 400.

In addition, the photographing device 200 may be installed near the upper portion of the outer surface of the door facing toward the interior of the building to photograph a user approaching from inside the building, and transmit the photographed image information to the control device 400.

The photographing device 200 can be implemented as various photographing modules such as a camera or CCTV, and can communicate with the control device 400 by being connected via a network.

In addition, a user may access the control device 400 via an application from a terminal carried by the user, and transmit user authentication information through the connected application. Such user authentication information may be transmitted together with the user authentication information registered in the user terminal when the application is launched or when registering to use the application.

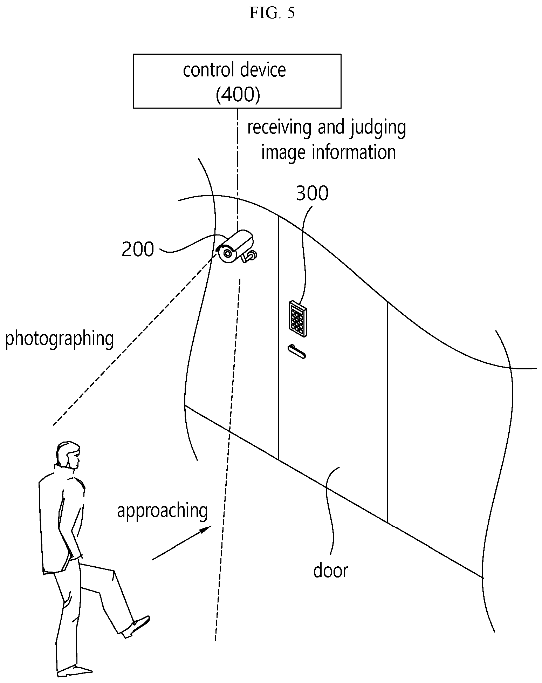

Returning to FIG. 3, a user approaching the door may then be determined based on the image information. <S310> This will be described with reference to FIG. 5.

FIG. 5 is a diagram exemplarily illustrating detecting a user and determining whether the user is an authenticated user in the apparatus for controlling opening and closing of a door according to an embodiment of the present invention.

As shown in FIG. 5, the control device 400 can detect and determine a user approaching the door based on image information photographed by the photographing device 200. The control device 400 can detect a user from the image information and determine whether the detected user is an authenticated user.

Here, the control device 400 can be implemented as an artificial intelligence model to detect a user from image information and determine whether the detected user is an authenticated user.

First, the artificial intelligence model can learn to detect a user or person from image information by applying image information as an input variable and a user or person as an output variable, and to determine whether the detected user or person is an authenticated user.

For example, the artificial intelligence model can learn to detect the appearance of a person from a plurality of photographs including people in various poses, ethnicities, ages, hairstyles, wearing accessories, and with mobility aids, and the like.

As such, since the technology for detecting a person from image information is already well known, a detailed description thereof will be omitted.

At this time, the control device 400 can determine an authenticated user based on user information or user authentication information registered in the control device 400.

For example, a user can register biometric information, a personal photograph, information capable of identifying the user terminal or mobile device, a means embedded with an RFID tag, and the like in the control device 400.

Accordingly, the control device 400 can learn the appearance of a user based on photographs including the face, appearance, and other characteristics of the authenticated user, and determine whether the user in the corresponding image information is an authenticated user.

Alternatively, a signal transmitted from a user terminal or mobile device carried by the user may be received to determine whether the user is an authenticated user.

For example, a user can access the control device 400 via an application installed on a mobile device carried by the user to request door lock unlocking and door opening, and the control device 400 can determine whether the user is an authenticated user based on the signal received from the mobile device.

At this time, the signal received from the mobile device may be a signal including biometric information such as iris, fingerprint, or face registered in the mobile device and the control device 400, or may be a signal transmitted from a device such as a smart door key or wireless remote control. This may be a well-known technology generally used when subscribing to an application.

In addition, the signal received from the mobile device may include an authentication key registered in the mobile device, device authentication information, and the like.

Furthermore, if the user is a person with a disability, an RFID signal generated from a disability registration card embedded with an RFID tag or the like may be received to determine whether the user is an authenticated user.

Alternatively, the control device 400 may not be implemented with an artificial intelligence model, but may receive information associated with an artificial intelligence algorithm from a cloud server and apply it when performing the determination of identifying and authenticating a user.

That is, user information associated with a user may be uploaded to and stored in a cloud server. Accordingly, the control device 400 can receive user information from the cloud server and apply an artificial intelligence algorithm based on the received user information to determine an authenticated user.

Such a cloud server is a server related to hospital data, MyData, or public data, and the control device 400 can be linked with the cloud server to obtain information about authenticated users from the cloud server. Accordingly, the control device 400 can determine whether a specific user has a disability and what physical characteristics the user has.

In addition, after the control device 400 authenticates a user, the authentication history may be automatically deleted. However, whether to delete the user authentication history may be set to manual deletion rather than automatic deletion through the terminal owned by the user, and accordingly, the control device 400 can inquire of the user terminal about whether to delete the user authentication history.

Furthermore, when the door lock device 300 is detached or damaged, or when entry/exit records are utilized, the control device 400 can control a speaker, display, or the like built into the door lock device 300 to display the information, and can notify the user or administrator via email, SNS, or message.

Returning to FIG. 3, next, a door lock unlocking signal may be transmitted to the door lock device 300 in response to determining that the user is an authenticated user. <S320>

Specifically, if the user or person approaching from the front of the door is an authenticated user, the control device 400 can transmit a door lock unlocking signal to the door lock device 300 to release the lock of the door lock with respect to the door in order to open the door. Thereby, the door lock device 300 can release the lock of the door lock that is locking the door closed.

Here, the door lock device 300 may be installed on any one part of the door. Accordingly, when in a locking operation, the door lock device 300 can connect the door and the wall of the building with at least one bar between the door and the wall of the building, and when unlocking, the door lock device 300 can disconnect at least one bar between the door and the wall of the building.

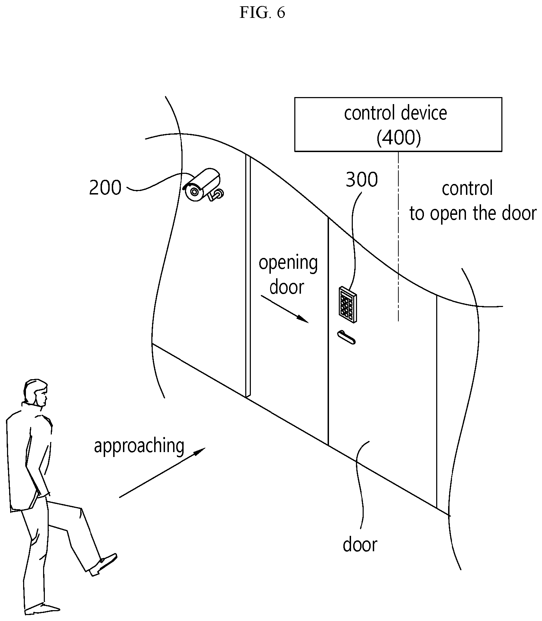

Returning to FIG. 3, finally, a door opening signal may be transmitted to the door opening/closing device 100 as the door lock device 300 releases the lock of the door in response to the door lock unlocking signal. <S330> This will be described with reference to FIG. 6.

FIG. 6 is a diagram exemplarily illustrating determining an authenticated user, releasing the door lock, and opening the door in the apparatus for controlling opening and closing of a door according to an embodiment of the present invention.

As shown in FIG. 6, after transmitting the door lock unlocking signal to the door lock device 300, the control device 400 can receive a signal from the door lock device 300 indicating that the unlocking of the door lock is complete, and then transmit a door opening signal to the door opening/closing device 100. Accordingly, the door can be opened so that the authenticated user can enter the building through the door.

Furthermore, when the entry of the authenticated user through the door is confirmed via the door opening/closing device 100, the door opening/closing device 100 can transmit to the control device 400 that the authenticated user has entered through the door.

Here, at least one sensor (not shown) installed on one side of the door is installed, and the sensor can detect an authenticated user entering or exiting through the door and transmit it to the door opening/closing device 100. Accordingly, the door opening/closing device 100 can transmit to the control device 400 that the authenticated user has passed through the door based on the signal received from the sensor.

In addition, after the authenticated user passes through the door, the control device 400 can transmit a door closing signal to the door opening/closing device 100 to control the door to close.

Furthermore, when the control device 400 receives a signal indicating that the closing of the door is complete, the control device 400 can transmit a door locking signal to the door lock device 300 to control the door lock to lock the door again. Accordingly, the door can be closed and locked.

Alternatively, in the absence of a sensor installed at the door, the control device 400 can also control the door to automatically close after a predetermined amount of time has elapsed after the door is opened.

Through such a series of processes, the control device 400 can detect a user approaching the door and control the door lock device 300 and the door opening/closing device 100 according to whether the detected user is an authenticated user, thereby providing only authenticated users with access to enter the interior of the building or exit to the exterior of the building through the door.

### <Control of Opening and Closing Speed of the Door>

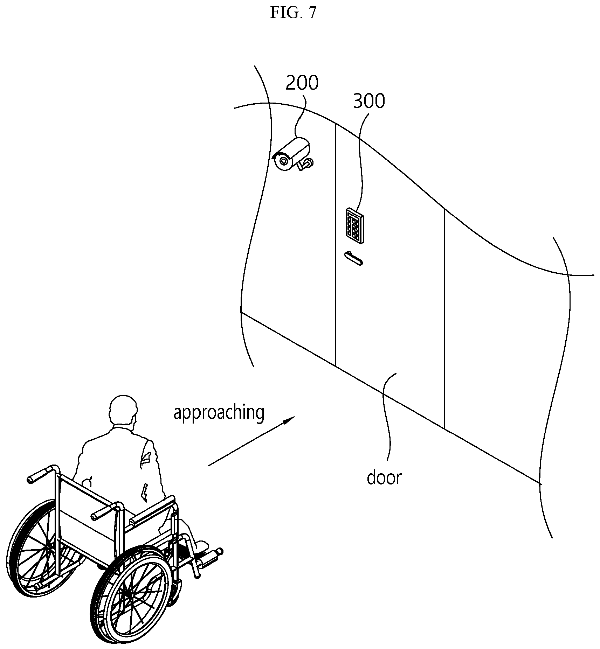

Hereinafter, the control device 400 can control the door to open not only when an authenticated user is detected but also when a person with a disability is detected. This will be described with reference to FIG. 7.

FIG. 7 is a diagram exemplarily illustrating detection of a person with a disability and control to open the door in the apparatus for controlling opening and closing of a door according to an embodiment of the present invention.

As shown in FIG. 7, when a user approaching the door is a person with a disability, the control device 400 can control the door to open by omitting a separate authentication procedure.

For example, a person with a disability may be a user with a leg impairment, that is, a person with a walking disorder. Here, a user with a leg impairment may be a user who limps due to impairment in one leg, or a user who moves with crutches.

Accordingly, when the control device 400 detects from image information that a user with a leg impairment is approaching the door, the control device 400 can control the door lock device 300 and the door opening/closing device 100 to open the door, thereby allowing the user with a leg impairment to enter the building.

In addition, a person with a disability may be a visually impaired person who cannot see. In this case, the visually impaired person may approach the door accompanied by a guide dog. Accordingly, when the control device 400 detects from image information that a visually impaired person accompanied by a guide dog is approaching the door, the control device 400 can control the door lock device 300 and the door opening/closing device 100 to open the door, thereby allowing the visually impaired person and the guide dog to enter the building.

Furthermore, a person with a disability may be a user riding a mobility aid such as a wheelchair, that is, a user approaching the door in a wheelchair. Accordingly, when the control device 400 detects from image information that a user is approaching the door in a wheelchair, the control device 400 can control the door lock device 300 and the door opening/closing device 100 to open the door, thereby allowing the user riding the mobility aid to enter the building.

Here, the control device 400 can differently control the angle and speed at which the door opens and closes according to each disability type.

Specifically, the control device 400 can classify users into visually impaired persons, persons with leg impairments, persons riding mobility aids, and the like, and can differently control the angle and speed at which the door opens and closes according to the classified disability type.

For example, in the case of a visually impaired person, the control device 400 can control the door to slowly open and slowly close at a first speed. In addition, since a visually impaired person and a guide dog may walk irregularly, such as one behind the other or at an angle, sufficient lateral space is required. Accordingly, the control device 400 can control the door opening/closing device 100 so that the door opens to a first angle of approximately 120 degrees, taking the closed state of the door as a reference of 0 degrees. Thus, the visually impaired person and the guide dog can pass through the door with sufficient space.

In the case of a person with a leg impairment, the control device 400 can control the door to open and close at a second speed, which is slower than the first speed. In addition, since a person with a leg impairment walks at a consistent pace, for example with crutches, the control device 400 can control the door opening/closing device 100 so that the door opens to a second angle of approximately 90 degrees. Accordingly, the person with a leg impairment can be controlled to pass through the door with sufficient space.

Furthermore, in the case of a person with a disability riding a mobility aid such as a wheelchair, the control device 400 can control the door to open at a third speed, which is faster than the first speed, and to close quickly, so that the person riding the mobility aid can pass through the door quickly and the door closes quickly thereafter.

In addition, since mobility aids such as wheelchairs occupy a large area, the control device 400 can control the door opening/closing device 100 so that the door opens to a third angle of approximately 180 degrees.

In this manner, the control device 400 can differently control the angle and speed at which the door opens and closes according to the disability type of the user included in the image information, thereby controlling the door so that the user can easily enter and exit through the door.

Of course, in the present invention, a user can also manually open the door. To this end, a button linked to the control device 400 may be provided on one side of the door. Accordingly, an authenticated user or a person with a disability can press the button to release the door lock and open the door.

Meanwhile, in the present invention, not only persons with disabilities but also persons without disabilities who are in an inconvenient state where they cannot use their hands - that is, a state where both hands of the user are occupied by a specific object and thus the door cannot be opened - the control device 400 can release the door lock and control the door to open.

For example, a state where a person cannot use their hands may be a person pushing a stroller. Accordingly, the control device 400 performs learning to detect a person pushing a stroller from image information, and upon detecting a person pushing a stroller from image information photographed around the door, transmits a door lock unlocking signal to the door lock device 300 to release the door lock, and transmits a door opening signal to the door opening/closing device 100 to control the door to open.

In addition, a state where a person cannot use their hands may be a state where the person is carrying luggage in both hands. Accordingly, the control device 400 performs learning to detect a person carrying luggage in both hands from image information, and upon detecting a person carrying luggage in both hands from image information photographed around the door, transmits a door lock unlocking signal to the door lock device 300 to release the door lock, and transmits a door opening signal to the door opening/closing device 100 to control the door to open.

In this manner, when not only persons with disabilities but also persons without disabilities are detected to be in an inconvenient state where they cannot use their hands, the control device 400 can release the door lock and control the door to open by controlling the angle and speed of the door, thereby allowing persons to easily enter and exit through the door.

The preferred embodiments of the present invention described above have been disclosed for illustrative purposes, and those skilled in the art having ordinary knowledge of the present invention will be able to make various modifications, changes, and additions within the spirit and scope of the present invention, and such modifications, changes, and additions should be considered to fall within the scope of the claims of the present invention.

## Claims

1. A method for controlling opening and closing of a door, comprising:
receiving at least one of image information generated by a photographing device and user authentication information around the door;
determining a user approaching the door based on at least one of the image information and the user authentication information;
transmitting a door lock unlocking signal to a door lock device in response to determining that the user is an authenticated user; and
transmitting a door opening signal to a door opening/closing device as the door lock device releases the lock of the door in response to the door lock unlocking signal.

2. The method of claim 1, wherein the determining of the user approaching the door comprises:
detecting, based on an artificial intelligence model, the user approaching the door from the image information; and determining whether the detected user is an authenticated user.

3. The method of claim 1, wherein the determining of the user approaching the door comprises:
detecting, based on an artificial intelligence model, the user approaching the door from the image information; and determining, based on the image information, a state associated with the detected user.

4. The method of claim 3, further comprising:
controlling the door opening/closing device so that the door opens and closes differently based on the state associated with the user.

5. The method of claim 4, wherein the state associated with the user includes at least one disability type, and
the door opening/closing device differently controls a speed and angle at which the door opens and closes based on the user corresponding to the at least one disability type.

6. The method of claim 5, wherein the at least one disability type includes a first type through a third type,
the first type controls the door opening/closing device to open and close the door at a first speed,
the second type controls the door opening/closing device to open the door more slowly than the first speed and to close the door more slowly than the first speed, and
the third type controls the door opening/closing device to open the door more quickly than the first speed and to close the door more quickly than the first speed.

7. The method of claim 5, wherein the at least one disability type includes a first type through a third type,
the first type controls the door opening/closing device to open the door to a first angle,
the second type controls the door opening/closing device to open the door to a second angle greater than the first angle, and
the third type controls the door opening/closing device to open the door to a third angle greater than the second angle.

8. The method of claim 5, wherein the disability type includes at least one of a visually impaired person, a person with a disability riding a mobility aid, and a person with a disability using crutches.

9. The method of claim 4, wherein the state associated with the user includes a state in which both hands of the user are occupied by a specific object, and
in response to determining from the image information that the user is in a state where it is impossible to use their hands, the door lock device is unlocked and the door opening/closing device is controlled to open the door.

10. The method of claim 1, wherein the user authentication information includes at least one of biometric information associated with the user registered in a user terminal, an authentication key, device authentication information, and an RFID signal.

11. The method of claim 1, wherein the transmitting of the door opening signal to the door opening/closing device as the door lock device releases the lock of the door further comprises:
transmitting the door opening signal to the door opening/closing device after receiving a signal from the door lock device indicating that the unlocking of the door lock is complete.

12. The method of claim 1, further comprising:
transmitting a door closing signal for closing the door to the door opening/closing device in response to detecting that the user has entered or exited through the door after the door is opened.

13. The method of claim 12, further comprising:
transmitting a door locking signal for switching the door to a locked state to the door lock device in response to detecting that the door is closed.

14. An apparatus for controlling opening and closing of a door, comprising:
an information transceiver configured to receive at least one of image information generated by a photographing device around the door and user authentication information;
a user authentication unit configured to determine a user approaching the door based on at least one of the image information and the user authentication information; and
a controller configured to transmit a door lock unlocking signal to a door lock device in response to determining that the user is an authenticated user, and to transmit a door opening signal to a door opening/closing device as the door lock device releases the lock of the door in response to the door lock unlocking signal.

15. A system for controlling opening and closing of a door, comprising:
a photographing device located around the door and configured to photograph a user approaching the door;
a door opening/closing device connected to the door and configured to open and close the door;
a door lock device installed on one side of the door and configured to lock or unlock the door; and
a control device configured to receive at least one of image information generated by the photographing device and user authentication information, determine a user approaching the door based on at least one of the image information and the user authentication information, transmit a door lock unlocking signal to the door lock device in response to determining that the user is an authenticated user, and transmit a door opening signal to the door opening/closing device as the door lock device releases the lock of the door in response to the door lock unlocking signal.
